# EUROPEAN PATENT SPECIFICATION

(11) **EP 2 888 231 B1**
(45) Date of publication and mention of the grant of the patent: **09.01.2019**
(21) Application number: 13837271.9
(22) Date of filing: 23.08.2013
(51) Int. Cl.: C07D 213/61, A61K 31/44, A61K 31/444, A61P 29/00

(54) **PROCESS FOR PREPARATION OF CRYSTALLINE ETORICOXIB**
VERFAHREN ZUR HERSTELLUNG VON KRISTALLINEM ETORICOXIB
PROCÉDÉ DE PRÉPARATION D'ÉTORICOXIB CRISTALLIN

(30) Priority: 27.08.2012 IN MM24802012; 10.10.2012 US 201261712069 P
(43) Date of publication of application: 01.07.2015
(73) Proprietor: Glenmark Pharmaceuticals Limited, Mumbai 400 099 (IN)
(72) Inventor: BHIRUD, Shekhar Bhaskar, Mumbai 400053 (IN); DSOUZA, Francis P, Navi Mumbai 400703 (IN); NAYKODI, Sachin B, Navi Mumbai 400705 (IN)
(74) Representative: HGF Limited
(86) International application number: PCT/IN2013/000513
(87) International publication number: WO 2014/041558

(56) References cited:
- WO-A1-2011/158250
- WO-A1-2012/163839
- WO-A1-2013/105106
- CN-A- 1 443 168
- US-B1- 6 858 631
- XING WANG ET AL: "Low Molecular Weight Poly(ethylene glycol) as an Environmentally Benign Solvent for Pharmaceutical Crystallization and Precipitation", CRYSTAL GROWTH & DESIGN., vol. 5, no. 1, 1 January 2005 (2005-01-01) , pages 85-92, XP055242145, US ISSN: 1528-7483, DOI: 10.1021/cg034208i
- SUHAGIAB.N. ET AL.: 'Prearation and characterization of etoricorization of etoricoxib-polyethylene glycol 4000 plus polyvinylpyrrolidone K30 solid dispersions' ACTA PHARM vol. 56, 2006, pages 285 - 298, XP008178515
- Chad R. Dalton ET AL: "Investigating the hydrate conversion propensity of different etoricoxib lots", Journal of Pharmaceutical Sciences, vol. 95, no. 1, 1 January 2005 (2005-01-01), pages 56-69, XP055033301, ISSN: 0022-3549, DOI: 10.1002/jps.20499

## Description

### PRIORITY

This application claims the benefit of IN2480/MUM/2012, filed on August 27, 2012, and United States Provisional Application No. 61/712,069, filed October 10, 2012.

### FIELD OF THE INVENTION

The present invention relates to a process for the preparation of crystalline etoricoxib.

### BACKGROUND OF THE INVENTION:

Selective inhibitors of cyclooxygenase-2 are a sub-class of drugs known as non-steroidal anti-inflammatory drugs (NSAIDs). Conventional NSAIDs block both forms of the cyclooxygenase (COX) enzyme and although active in reducing pain and swelling associated with the inflammatory process, can produce severe side effects. The identification of the COX-2 enzyme associated with inflammation has provided a viable target of inhibition which more effectively reduces inflammation and produces fewer and less drastic side effects. The selective COX-2 inhibitors (coxibs) were originally developed to minimize the adverse effects of conventional non-steroidal anti-inflammatory drugs (NSAIDs) while maintaining the same analgesic and anti-inflammatory properties.

Etoricoxib is a coxib, which is a class of arthritis/analgesia medications. Etoricoxib has been approved in Europe and is sold under the brand name Arcoxia®. Etoricoxib is chemically designated as 5-chloro-3-[4-methylsulfonyl)phenyl]-2-(2-methyl-5-pyridinyl) pyridine with the structural formula:

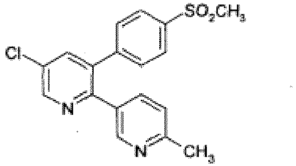

Etoricoxib is known to exist in various polymorphic forms for example form I, form II, form III, form IV, form V, amorphous form and hydrated form.

United States Patent No 6858631 (US'631) discloses polymorphic form I of etoricoxib characterized by powder X-ray diffraction (PXRD) pattern peak at 9.7 degrees 2 theta. US'631 further discloses form II of etoricoxib characterized by differential scanning calorimetry having an extrapolated onset of melting at 131±1°C and a peak melting point of 132.5±1°C.

United States Patent No 6441002 (US'002) discloses polymorphic form III, form IV, hemihydrate form and amorphous form of etoricoxib. Polymorphic form III was characterized by powder X-ray diffraction (PXRD) peak at 10.5, 16.1 or 22.4 degrees 2 theta. Polymorphic form IV was characterized by powder X-ray diffraction (PXRD) peak at 8.7 and 15.2. US '002 teaches hemihydrate form containing about 0.5 water per mol of etoricoxib.

European Patent No. 1296951B1 discloses form V of etoricoxib characterized by powder X-ray diffraction (PXRD) peak position at 13.7 angstroms.

It has been observed that crystalline etoricoxib characterized by PXRD having peak reflections at about 7.1, 9.7, 11.8, 15.5 and 22.7 ±0.2 degrees 2 theta, prepared according to known methods, on storage, gets converted to hemihydrate form, form IV, form V or mixtures thereof and is thus unstable.

Herein is described a process for the preparation of a stable crystalline etoricoxib with peak reflections at about 7.1, 9.7, 11.8, 15.5 and 22.7 ± 0.2 degrees 2 theta, having no or little tendency to convert to any of the other polymorphic forms. The crystalline etoricoxib prepared according to the process of the present invention, remains stable on storage.

### SUMMARY OF THE INVENTION

In a first aspect the present invention provides a process for the preparation of crystalline etoricoxib, characterized by powder X-ray diffraction (PXRD) pattern having peak reflections at about 7.1, 9.7, 11.8, 15.5 and 22.7 ± 0.2 degrees 2 theta comprising:
a) treating etoricoxib with a solvent system comprising at least one solvent selected from the group consisting of glycols, polyalkylene glycols and mixtures thereof; and
b) isolating crystalline etoricoxib.

In a second aspect the present invention provides a stable crystalline form of etoricoxib obtainable by the process according to any one of claims 1 to 8 characterized by PXRD pattern having peak reflections at about 7.1, 9.7, 11.8, 15.5 and 22.7 ± 0.2 degrees 2 theta, wherein the stable crystalline form of etoricoxib has no detectable quantity of any of form IV, form V or hemihydrate form of etoricoxib on storage at about 25°C and about 60% relative humidity for at least two months.

### BRIEF DESCRIPTION OF THE ACCOMPANYING FIGURES

Fig.1: PXRD of crystalline etoricoxib, which is substantially in accordance with Ex.1.
Fig.2: PXRD of etoricoxib hemihydrate, which is substantially in accordance with reference Ex.1.

### DETAILED DESCRIPTION OF THE INVENTION

The present invention provides a process for the preparation of crystalline etoricoxib, characterized by PXRD pattern having peak reflections at about 7.1, 9.7, 11.8, 15.5 and 22.7 ±0.2 degrees 2 theta comprising:
a) treating etoricoxib with a solvent system comprising at least one solvent selected from the group consisting of glycols, polyalkylene glycols and mixtures thereof; and
b) isolating crystalline etoricoxib.

In one embodiment, the present invention provides a process for the preparation of crystalline etoricoxib, characterized by PXRD pattern having peak reflections at about 7.1, 9.7, 11.8, 15.5, 18.1, 20.1, 22.7, 24.1 and 29.2 ±0.2 degrees 2 theta comprising :
a) treating etoricoxib with a solvent system comprising at least one solvent selected from the group consisting of glycols, polyalkylene glycols and mixtures thereof; and
b) isolating crystalline etoricoxib.

As used herein, "glycol" refers to an organic compound in which two hydroxyl groups are attached to different carbon atoms and includes ethylene glycol, propylene glycol, butylene glycol.

As used herein, "polyalkylene glycol" includes compounds such as polyethylene glycol (PEG), polypropylene glycol, polybutylene glycol and the like. Preferably, the polyalkylene glycol may be PEG having molecular weight range 200, 400, 800, 900, 1000, 1200, 2000 and 4000. More preferably the PEG may be PEG-400.

As used herein, "treating" refers to contacting, heating, crystallizing, slurrying, etoricoxib in a solvent system comprising at least one solvent selected from the group consisting of glycols and polyalkylene glycols.

In one embodiment, the solvent system comprises alcohols, nitriles, ketones, esters and at least one of glycols, polyalkylene glycols and mixtures thereof.

The alcohol may be selected from C1-C5 alcoholic solvent group consisting of methyl alcohol, ethyl alcohol, propyl alcohol, isopropyl alcohol, butyl alcohol, tertiary butyl alcohol, isobutyl alcohol and the like. Preferably the alcohol may be isopropyl alcohol or its mixture with other alcohols.

Ketones may be selected from the group consisting of acetone, methyl ethyl ketone, methyl isobutyl ketone and the like.

Esters may be selected from the group consisting of isopropyl acetate, ethyl acetate, butyl acetate and the like.

Nitriles may be selected from the group consisting of acetonitrile, propionitrile, butyronitrile and the like.

In one embodiment, the present invention provides a process for the preparation of crystalline etoricoxib characterized by PXRD pattern having peak reflections at about 7.1, 9.7, 11.8, 15.5 and 22.7 ±0.2 degrees 2 theta, said process comprising treating etoricoxib with a solvent system comprising a C1-C5 alcohol and at least one solvent selected from the group consisting of glycols and polyalkylene glycols and isolating crystalline etoricoxib.

In one embodiment the solvent system comprises C1-C5 alcoholic solvent and polyalkylene glycol in the ratio of about 0.25: 550 by volume to about by 2.5:170 by volume. Preferably the ratio of C1-C5 alcoholic solvent and polyalkylene glycol is 0.5:450 by volume.

In one embodiment, the present invention provides a process for the preparation of crystalline etoricoxib characterized by PXRD pattern having peak reflections at about 7.1, 9.7, 11.8, 15.5 and 22.7±0.2 degrees 2 theta, the process comprising treating etoricoxib with a solvent system comprising C1-C5 alcohol and polyethylene glycol and isolating crystalline etoricoxib.

In one embodiment, the present invention provides a process for the preparation of crystalline etoricoxib characterized by PXRD pattern having peak reflections at about 7.1, 9.7, 11.8, 15.5 and 22.7±0.2 degrees 2 theta, the process comprising crystallizing etoricoxib from a solvent system comprising isopropyl alcohol and PEG-400 and isolating crystalline etoricoxib.

In one embodiment, the present invention provides a process for the preparation of crystalline etoricoxib, as described herein above, the process comprising crystallizing etoricoxib by heating a mixture of etoricoxib, a C1-C5 alcohol and PEG-400 to a temperature of about 60-80°C, preferably 70-75°C; cooling and isolating crystalline etoricoxib by techniques known in the art. These isolation techniques include for example filtration, centrifugation, evaporation and the like.

In one embodiment, the present invention provides a process for preparation of stable crystalline etoricoxib, the process comprising heating a mixture of isopropyl alcohol, etoricoxib and PEG-400 to a temperature of about 60-85°C. Preferably, the mixture is heated to a temperature of about 70-75°C; stirring the resultant hot mixture until a clear solution is obtained; optionally, treating the hot solution containing etoricoxib with charcoal and filtering through hyflo; gradually cooling the hot solution . The cooling is preferably carried out under nitrogen atmosphere. The reaction mass is maintained at a temperature of about -5 to about 10 °C. Preferably, the reaction mass is maintained at a temperature of about 0-5°C. Crystalline etoricoxib thus formed is isolated by filtration. The wet cake of crystalline etoricoxib is dried under vacuum in a rotary cone vacuum drier for a period of about 15 to 33 hours. Preferably etoricoxib is dried under vacuum in a rotary vacuum drier for a period of about 18 to 23 hours. The dried etoricoxib is subjected to milling and sifting and packed in polyethylene bags and silica gel pouches are kept in between white and black polyethylene bags. This is further sealed with a polyvinyl chloride (PVC) strip and placed in triple laminated aluminium bags and the aluminium bags are sealed.

In one embodiment, the present invention provides a process for preparing crystalline etoricoxib, characterized by PXRD pattern having peak reflections at about 7.1, 9.7, 11.8, 15.5, 18.1, 20.1, 22.7, 24.1 and 29.2 ±0.2 degrees 2 theta, the process comprising :
a) crystallizing etoricoxib with a solvent system comprising a C1-C5 alcoholic solvent and polyalkylene glycol; and
b) isolating crystalline etoricoxib.

The C1-C5 alcoholic solvent and polyalkylene glycol may be selected from the group as disclosed earlier.

The polymorphic form IV, form V and hemihydrate form of etoricoxib are known in the art. Illustratively, form IV and hemihydrate are disclosed in EP1248618B1 (EP'618) and form V is disclosed in EP1296951B1 (EP'951) and are included by reference herein in its entirety. Polymorphic form IV and form V are characterized by XRD peaks in EP'618 and EP'915 respectively. The XRD pattern of hemihydrate is disclosed in Fig. 5 of EP'618.

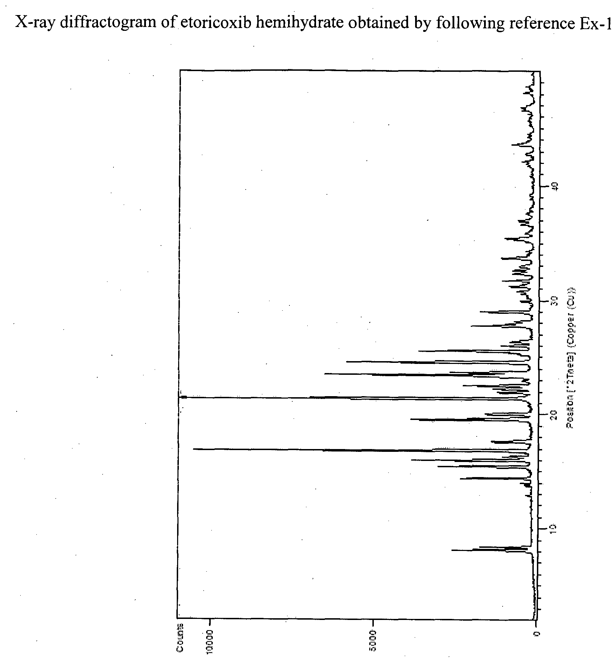

In one embodiment, etoricoxib hemihydrate was prepared by a process comprising addition of water to a solution of etoricoxib in acetone. The product was isolated and dried to obtain etoricoxib hemihydrate. The etoricoxib hemihydrate thus obtained is characterized by peak reflection at about 8.1±0.2 degrees 2 theta and an XRD pattern which is substantially in accordance with Fig. 2 and matches with that reported in Fig. 5 of EP'618.

In one embodiment, the present invention provides a process for the preparation of crystalline etoricoxib having characteristic PXRD peak reflections at about 7.1, 9.7, 11.8, 15.5 and 22.7±0.2 degrees 2 theta, wherein the crystalline form of etoricoxib has no detectable quantity of form IV as determined by absence of characteristic peak reflections at about 8.7±0.2 degrees 2 theta.

In one embodiment, the present invention provides a process for the preparation of crystalline etoricoxib having characteristic PXRD peak reflections at about 7.1, 9.7, 11.8, 15.5 and 22.7±0.2 degrees 2 theta, wherein the stable crystalline form of etoricoxib has no detectable quantity of form IV as determined by absence of characteristic peak reflections at about 8.7 and 15.2±0.2 degrees 2 theta.

In one embodiment, the present invention provides process for the preparation of crystalline etoricoxib having characteristic PXRD peak reflections at about 7.1, 9.7, 11.8, 15.5 and 22.7±0.2 degrees 2 theta, wherein the stable crystalline form of etoricoxib has no detectable quantity of form V as determined by absence of characteristic peak reflections at about 13.7±0.2 degrees 2 theta.

In one embodiment, the present invention provides process for the preparation of crystalline etoricoxib having characteristic PXRD peak reflections at about 7.1, 9.7, 11.8, 15.5 and 22.7±0.2 degrees 2 theta, wherein the stable crystalline form of etoricoxib has no detectable quantity of form V as determined by absence of characteristic peak reflections at about 6.5 and 17.7±0.2 degrees 2 theta.

In one embodiment, the present invention provides process for the preparation of crystalline etoricoxib having characteristic PXRD peak reflections at about 7.1, 9.7, 11.8, 15.5 and 22.7±0.2 degrees 2 theta, wherein the stable crystalline form of etoricoxib has no detectable quantity of hemihydrate as determined by absence of characteristic peak reflections at about 8.1 ± 0.2 degrees 2 theta.

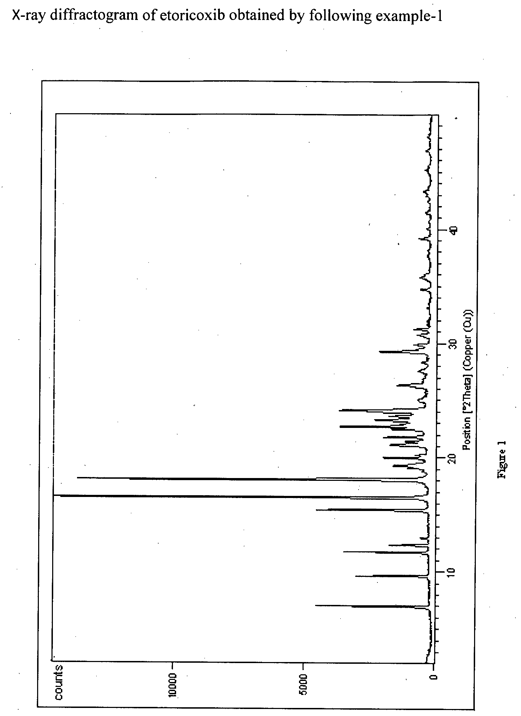

In one embodiment, the present invention provides a stable crystalline form of etoricoxib characterized by PXRD pattern, which is substantially in accordance with Fig 1.

Herein is described a stable crystalline form of etoricoxib characterized by PXRD pattern with peaks at about 7.1, 9.7, 11.8, 15.5 and 22.7 ± 0.2 degrees 2 theta.

In one embodiment, the present invention provides stable crystalline form of etoricoxib characterized by PXRD pattern with peaks at about 7.1, 9.7,
11.8, 18.8, 15.5, 20.1, 22.7, 24.1 and 29.2 ± 0.2 degrees 2 theta.

As used herein, the term "stable" refers to crystalline etoricoxib, characterized by PXRD having peak reflections at about 7.1, 9.7, 11.8, 15.5 & 22.7±0.2 degrees 2 theta, which retains its original polymorphic form without undergoing polymorphic conversion over time.

Herein is described a stable crystalline etoricoxib obtained by the process as described herein above having characteristic PXRD peak reflections at about 7.1, 9.7, 11.8, 15.5 and 22.7 ± 0.2 degrees 2 theta and has no detectable quantity of any form IV, form V or hemihydrate form of etoricoxib on storage.

Herein is described a stable crystalline etoricoxib obtained by the process as disclosed above having characteristic PXRD peak reflections at about 7.1, 9.7, 11.8, 15.5 and 22.7 ± 0.2 degrees 2 theta and has no detectable quantity of any form IV, form V or hemihydrate form of etoricoxib on storage.

Herein is described a stable crystalline etoricoxib characterized by PXRD having peak reflections at about 7.1, 9.7, 11.8, 15.5 and 22.7 ± 0.2 degrees 2 theta, whereing stable crystalline form of etoricoxib has no detectable quantity of any of form IV, form V or hemihydrate form of etoricoxib at room temperature or under accelerated stability conditions for extended periods of time.

The present invetion provides a stable crystalline etoricoxib characterized by PXRD pattern having peak reflections at about 7.1, 9.7, 11.8, 15.5 and 22.7 ± 0.2 degrees 2 theta, wherein stable crystalline form of etoricoxib has no detectable quantity
of any of form IV, form V or hemihydrate form of etoricoxib on storage at about 25°C for a period of about two years.

Herein is described a stable crystalline etoricoxib, characterized by PXRD pattern having peak reflections at about 7.1, 9.7, 11.8, 15.5 and 22.7 ± 0.2 degrees 2 theta, wherein stable crystalline form of etoricoxib has no detetable quantity of any of form IV, form V or hemihydrate form of etoricoxib on storage at about 2-8°C.

The present invention provides a stable crystalline etoricoxib, characterized by PXRD pattern having peak reflections at about 7.1, 9.7, 11.8, 15.5 and 22.7 ± 0.2 degrees 2 theta, wherein stable crystalline form of etoricoxib has no detetable quantity of any of form IV, form V or hemihydrate form of etoricoxib on storage at about 25°C and about 60% relative humidity (RH) for at least 2 months.

Herein is described a stable crystalline etoricoxib, characterized by PXRD pattern having peak reflections at about 7.1, 9.7, 11.8, 15.5 and 22.7 ± 0.2 degrees 2 theta, wherein stable crystalline form of etoricoxib has no detetable quantity of any of form IV, form V or hemihydrate form of etoricoxib on storage at about 30°C and about 65%RH for at least 2 months.

Herein is described a stable crystalline form of etoricoxib characterized by PXRD pattern having peak reflections at about 7.1, 9.7, 11.8, 15.5 and 22.7 ± 0.2 degrees 2 theta, wherein stable crystalline form of etoricoxib has no detetable quantity of any of form IV, form V or hemihydrate form of etoricoxib on storage at about 40°C and about 75% relative humidity for a period of about six months.

The term "no detectable quantity" refers to an amount of less than about 0.5% w/w. Perferably, less than about 0.1%w/w, still more prefereably, absent.

Herein is described a stable crystalline form of etoricoxib characterized by PXRD pattern having peak reflections at about 7.1, 9.7, 11.8, 15.5 and 22.7 ± 0.2 degrees 2 theta, having no or little tendency to convert to any of the other polymorphic forms or solvates including hydrates.

In one embodiment, the stable crystalline form of etoricoxib of the present invention contains less than about 2% w/w of form II, contains less than about 1 % w/w of form II. Preferably, the stable crystalline etoricoxib of the present invention has no detectable quantity of form II.

In one embodiment, the stable crystalline form of etoricoxib of the present invention contains less than about 2% w/w of form III, contains less than about 1% w/w of form III. Preferably, the stable crystalline etoricoxib of the present invention has no detectable quantity of form III.

In one embodiment, the stable crystalline form of etoricoxib of the present invention contains less than about 2% w/w of form IV, contains less than about 1 w/w % of form IV. Preferably, the stable crystalline etoricoxib of the present invention has no detectable quantity of form IV.

In one embodiment, the stable crystalline form of etoricoxib of the present invention contains less than about 2% w/w of form V, contains less than about 1 % w/w of form V. Preferably, the stable crystalline etoricoxib of the present invention has no detectable form V.

In one embodiment, the stable crystalline form of etoricoxib of the present invention contains less than about 2% w/w of hemihydrate form, contains less than, about 1% of hemihydrate form. Preferably, the stable crystalline etoricoxib of the present invention has no detectable quantity of hemihydrate form.

In one embodiment, the stable crystalline form of etoricoxib of the present invention contains less than about 2% w/w of any of form II, form III, form IV, form V or hemihydrate form, contains less than about 1% w/w of any of form II, form III, form IV, form V or hemihydrate form. Preferably, the stable crystalline form of etoricoxib of the present invention has no detectable quantity of any of form II, form III, form IV, form V or hemihydrate form.

In one embodiment, the present invention provides stable crystalline form of etoricoxib characterized by PXRD pattern having peak reflections at about 7.1, 9.7, 11.8, 15.5 and 22.7±0.2 degrees 2 theta wherein, the stable crystalline form of etoricoxib has no detectable quantity of form IV as determined by absence of characteristic peak reflections at about 8.7±0.2 degrees 2 theta.

In one embodiment, the present invention provides stable crystalline form of etoricoxib characterized by PXRD pattern having peak reflections at about 7.1, 9.7, 11.8, 15.5 and 22.7±0.2 degrees 2 theta wherein, the stable crystalline form of etoricoxib has no detectable quantity of form IV as determined by absence of characteristic peak reflections at about 8.7 and 15.2±0.2 degrees 2 theta.

In one embodiment, the present invention provides stable crystalline form of etoricoxib characterized by PXRD pattern having peak reflections at about 7.1, 9.7, 11.8, 15.5 and 22.7±0.2 degrees 2 theta wherein, the stable crystalline form of etoricoxib has no detectable quantity of form IV as determined by absence of characteristic peak reflections at about 13.7±0.2 degrees 2 theta.

In one embodiment, the present invention provides stable crystalline form of etoricoxib characterized by PXRD having peak reflections at about 7.1, 9.7, 11.8, 15.5 and 22.7±0.2 degrees 2 theta wherein, the stable crystalline form of etoricoxib has no detectable quantity of form V as determined by absence of characteristic peak reflections at about 6.5 and 17.7±0.2 degrees 2 theta.

In one embodiment, the present invention provides stable crystalline form of etoricoxib characterized by PXRD having peak reflections at about 7.1, 9.7, 11.8, 15.5 and 22.7±0.2 degrees 2 theta wherein, the stable crystalline form of etoricoxib has no detectable quantity of hemihydrate form as determined by absence of characteristic peak reflections at about 8.1±0.2 degrees 2 theta.

### Instrumental settings for XRPD of stable crystalline etoricoxib:

The measurements were performed on Philips X-Ray Diffractometer model XPERT-PRO (PANalytical) Detector: X'celerator [1] using Cu lamp with type and wavelength of the X-ray radiation: K-Alphal [A] and 1.54060 under the following conditions: Generator settings: 40mA/45kV, Time per step: 50, Step size: 0.0167, Peak width 2.00 and start angle (°) 2.0 and End angle: 50.0, Scan type: continuous; measurement performed at 25°C. The XRPD instrument is calibrated using NIST SRM 6-40C silicon standard and NIST SRM 1976 Alumina.

Sample preparation: Adequate amount of sample was taken and filled in the sample holder using back-loading technique. Then sample holder was loaded between X-ray optics-path and scan using the above mentioned instrumental parameters. Integrated the obtained powder X-ray diffraction profiles using X' pert high score software.

Etoricoxib of a defined particle size may be produced by recrystallization from various solvents. It is well accepted that particle size plays an important role in the solubility properties of an active pharmaceutical ingredient (API). Particle size reduction leads to increase in surface area of the solid phase that is in contact with the liquid phase, thus leading to increased solubility. Rate of dissolution of a poorly soluble drug may limit its rate of absorption, thus affecting its bioavailability. In such cases, particle size reduction may enhance the absorption thus improving the bioavailability. Further, particle size can also affect how freely crystals or a powdered form of a drug will flow past each other, which in turn, has consequences in the production process of pharmaceutical products containing the drug. In view of all these it is desirable to have a defined particle size for an API.

The various techniques employed to attain a defined particle size are well known in the art. These methods include pH adjustment, cooling, evaporation of solvent, addition of antisolvent to a solution or by co-precipitation to obtain a precipitate with a defined particle size. Particle size of etoricoxib may be further adjusted by employing known methods of particle size reduction like compaction, milling or micronizing and sorting the milled product according to particle size.

In one embodiment of the present invention provides a process for preparing etoricoxib having D₉₀ between 40-80 µ. The process involves feeding etoricoxb into a compactor wherein the hydraulic pressure is about 0.5 to about 0.8 ton. The compactor is set an RPM (rate per minute) of 3. Etoricoxib thus obtained are milled and finally sieved to get etoricoxib of the desired particle size.

In one embodiment of the present invention, the etoricoxib has D₁₀ of about 2.91, D₅₀ of about 15.68 and D₉₀ of about 55.9µ.

Particle size of etoricoxib was measured by Malvern Mastersizer 2000®.

In one embodiment the present invention relates to a pharmaceutical composition comprising a therapeutically effective amount of a storage stable crystalline etoricoxib.

The following examples are provided to enable one skilled in the art to practice the invention and are merely illustrative of the invention. The examples should not be read as limiting the scope of the invention as defined in the feature and advantages.

### EXAMPLES

### COMPARATIVE EXAMPLE 1

### Preparation of 5-chloro-3-[4-methylsulfonyl)phenyl]-2-(2-methyl-5-pyridinyl) pyridine, etoricoxib.

In a 250ml 4-neck round bottom flask equipped with overhead stirrer, double surface condenser, thermowell pocket placed in water bath, 135ml (3volume) of isopropyl alcohol was added followed by 45.0gm etoricoxib; then the reaction mass was heated to about 65°C to about 70°C. The reaction mass was charcoalised with NORIT® charcoal and hot filtered on a hyflo bed and washed with 45 ml (1 volume) hot isopropyl alcohol. The filtered mass was gradually cooled to about 25°C to about 30°C with continuous stirring and then further cooled to about 0°C to about 5°C and maintained for about an hour and then filtered and washed with 45ml (1 volume) isopropyl alcohol. The wet solids were dried in air oven. 38gm of etoricoxib was obtained.

### Reference Example 1: preparation of etoricoxib hemihydrate.

In a one litre 4-neck round bottom flask equipped with one condenser, dropping funnel, placed in a plastic bath was charged 25 gm etoricoxib and 125ml acetone. To this solution 250 ml water was added and the reaction mixture was stirred for about 2 hr at a temperature of about 25-30°C. the solid was filtered and dried to obtain etoricoxib hemihydrate.

**Table 1: Below table provides XRD of etoricoxib hemihydrate.**

| Pos. [°2Th.] | d-spacing [Å] | Rel. Int. [%] | Pos. [°2Th.] | d-spacing [Å] | Rel. Int.[%] | Pos. [°2Th.] | d-spacing [Å] | Rel. Int. [%] |
|---|---|---|---|---|---|---|---|---|
| 8.1 | 10.8 | 22.7 | 19.6 | 4.5 | 16.6 | 25.9 | 3.4 | 8.6 |
| 8.3 | 10.5 | 15.3 | 19.9 | 4.4 | 12.0 | 26.3 | 3.3 | 6.1 |
| 12.8 | 6.86 | 1.6 | 20.0 | 4.40 | 12.9 | 27.7 | 3.2 | 17.0 |
| 13.7 | 6.4 | 1.9 | 21.3 | 4.1 | 100.0 | 27.9 | 3.1 | 7.3 |
| 13.9 | 6.3 | 3.0 | 21.9 | 4.0 | 9.4 | 28.0 | 3.1 | 4.7 |
| 14.4 | 6.1 | 20.0 | 22.2 | 3.9 | 10.5 | 28.9 | 3.0 | 14.4 |
| 15.4 | 5.7 | 26.2 | 22.4 | 3.9 | 18.9 | 29.8 | 2.9 | 3.2 |
| 15.9 | 5.5 | 34.1 | 22.9 | 3.8 | 2.4 | 30.5 | 2.9 | 2.9 |
| 16.2 | 5.4 | 8.1 | 23.4 | 3.7 | 58.7 | 30.7 | 2.9 | 4.1 |
| 16.8 | 5.2 | 96.1 | 23.6 | 3.7 | 22.9 | 31.1 | 2.8 | 6.3 |
| 17.5 | 5.02 | 11.0 | 24.5 | 3.6 | 52.0 | 31.6 | 2.8 | 8.5 |
| 19.5 | 4.5 | 33.4 | 25.5 | 3.4 | 32.1 | | | |

### EXAMPLE 1

### Preparation of 5-chloro-3-[4-methylsulfonyl)phenyl]-2-(2-methyl-5-pyridinyl) pyridine, etoricoxib

56 gm of etoricoxib, 448 ml of isopropyl alcohol and 0.56 gm of PEG-400 were charged into a round bottom flask with stirring at about 25-30°C for about 5 minutes. The reaction mass was then heated to about 70-75°C and charcoal was added. The reaction mass was heated to a temperature of about 80-85°C. Stirring was continued for about 30 minutes to about 2 hours. Reaction mass was filtered through hyflo. The filtrate was stirred arid cooled to about 0-15°C for about 1 hour. The reaction mass, was filtered and washed with isopropyl alcohol and dried the solid crystalline etoricoxib at about 80-85°C. Yield: 48 gm; HPLC purity = 99.95%.

**Table 2: Below table provides XRD of crystalline etoricoxib.**

| Pos[ 2th] | Rel Int [%] | Pos[2th] | Rel Int [%] | Pos[2th] | Rel Int [%] | Pos[ 2th] | Rel Int [%] |
|---|---|---|---|---|---|---|---|
| 7.1 | 29.5 | 19.3 | 8.2 | 23.6 | 10.8 | 31.2 | 4.0 |
| 9.7 | 18 | 20.1 | 10.4 | 23.9 | 10.8 | 31.6 | 0.6 |
| 11.8 | 26.3 | 20.2 | 3.2 | 24.1 | 20.5 | 31.9 | 0.7 |
| 12.4 | 11.6 | 21.1 | 8.0 | 25.0 | 2.02 | 33.1 | 0.8 |
| 13.0 | 2.2 | 21.2 | 8.8 | 26.3 | 7.24 | 33.5 | 0.5 |
| 14.1 | 0.4 | 21.4 | 4.9 | 26.8 | 1.28 | 34.7 | 2.6 |
| 15.5 | 29.7 | 21.8 | 10.0 | 27.6 | 2.25 | 35.7 | 2.5 |
| 16.6 | 100 | 22.5 | 8.5 | 28.3 | 1.77 | 39.2 | 3.5 |
| 18.1 | 98.1 | 22.7 | 22.9 | 29.2 | 11.6 | 40.0 | 0.9 |
| 18.8 | 1.2 | 22.9 | 6.3 | 29.8 | 3.67 | 40.6 | 0.4 |
| 19.1 | 4.8 | 23.2 | 12.6 | 30.7 | 2.57 | | |

The X-ray diffraction pattern was obtained using an X-ray diffractometer (Philips X'pert pro, PANalytical). The crystalline etoricoxib as prepared above was filled into a sample holder using back-loading technique. The sample holder was loaded between the X-ray optics path and scanned.

### Stability Data

Crystalline etoricoxib was stored at 40°C and 75% relative humidity. The content of other polymorphic forms was measured at periodic intervals.

Table 3: Stability data of crystalline etoricoxib prepared by the present invention at periodic intervals.

**Table 1**

| Time of storage in months Polymorphic form | 15 days | | | 1 month | | | 2 month | | | 6 months | | |
|---|---|---|---|---|---|---|---|---|---|---|---|---|
| | form IV | form V | hemihydrate | form IV | form V | hemihydrate | form IV | form V | hemihydrate | form IV | form V | hemihydrate |
| Crystalline etoricoxib obtained from comparative example 1 | 0.42 | NA | 0.81 | 0.52 | NA | 0.9 | 1.43 | NA | 1.11 | NA | NA | NA |
| Stable crystalline etoricoxib obtained from example 1 | ND | ND | ND | ND | ND | ND | ND | ND | ND | ND | ND | ND |

| | | | | | | | | | | | | |
|---|---|---|---|---|---|---|---|---|---|---|---|---|
| *NA = Not applicable; ND = not detected | | | | | | | | | | | | |

The results indicate that the crystalline form of etoricoxib of the present invention is stable and does not undergo polymorphic conversion at 40°C and 75% relative humidity. In contrast, the samples prepared according to comparative example 1 resulted to a substantial increase in the presence of other polymorphic forms at the end of two months under similar conditions.

Table 4: The table below provides percentage contamination with other polymorphic form of crystalline etoricoxib obtained from comparative example 1.

**Table 2**

| Condition of storage | Time | | |
|---|---|---|---|
| | 15 days | 1 month | 2 month |
| 2-8°C | Not detected | 0.53 hemihydrate | 0.58 hemihydrate |
| 25°C at 60%RH | 0.52 hemihydrate | 0.55 hemihydrate | 0.65 hemihydrate |
| 30°C at 65% RH | 0.66 hemihydrate | 0.68 hemihydrate | 0.69 hemihydrate and 1.23 form IV |

The above results indicate that etoricoxib prepared according to the present invention is chemically and physically stable at room temperature and accelerated stability conditions.

## Claims

1. A process for the preparation of crystalline etoricoxib, **characterized by** PXRD pattern having peak reflections at 7.1, 9.7, 11.8, 15.5 and 22.7±0.2 degrees 2 theta comprising :
a) treating etoricoxib with a solvent system comprising at least one solvent selected from the group consisting of glycols, polyalkylene glycols and mixtures thereof; and
b) isolating the crystalline etoricoxib.

2. The process as claimed in claim 1, wherein the solvent system comprises C1-C5 alcoholic solvent wherein the solvent is selected from the group consisting of methanol, ethanol, isopropanol, n-propanol, n-butanol, isobutanol, tertiary-butanol and mixtures thereof.

3. The process as claimed in claim 1, wherein the solvent system comprises C1-C5 alcoholic solvent and polyalkylene glycol in the ratio of 0.25:550 by volume to 2.5: 170 by volume.

4. The process as claimed in claim 1, wherein the treatment comprises crystallizing etoricoxib from a solvent system comprising C1-C5 alcoholic solvent and polyalkylene glycol.

5. The process as claimed in claim 1, wherein the crystalline etoricoxib obtained has characteristic PXRD pattern having peak reflections at 7.1, 9.7, 11.8, 15.5 and 22.7 ±0.2 degrees 2 theta and has no detectable quantity of any of form IV, form V or hemihydrate form of etoricoxib.

6. The process as claimed in claim 1, wherein the obtained crystalline etoricoxib has characteristic PXRD pattern having peak reflections at 7.1, 9.7, 11.8, 15.5 and 22.7 ±0.2 degrees 2 theta, wherein the stable crystalline form of etoricoxib has no detectable quantity of form IV as determined by absence of characteristic peak reflections at 8.7 and 15.2±0.2 degrees 2 theta.

7. The process as claimed in claim 1, wherein the crystalline etoricoxib obtained has characteristic PXRD pattern having peak reflections at 7.1, 9.7, 11.8, 15.5 and 22.7±0.2 degrees 2 theta, wherein the stable crystalline form of etoricoxib has no detectable quantity of form V as determined by absence of characteristic peak reflections at 13.7±0.2 degrees 2 theta or at 6.5 and 17.7±0.2 degrees 2 theta.

8. The process as claimed in claim 1, wherein the crystalline etoricoxib obtained has characteristic PXRD pattern having peak reflections at 7.1, 9.7, 11.8, 15.5 and 22.7 ±0.2 degrees 2 theta, wherein the stable crystalline form of etoricoxib has no detectable quantity of hemihydrate form as determined by absence of characteristic peak reflections at 8.1±0.2 degrees 2 theta.

9. A stable crystalline form of etoricoxib obtainable by a process according to any one of claims 1 to 8, **characterized by** PXRD pattern having peak reflections at 7.1, 9.7, 11.8, 15.5 and 22.7±0.2 degrees 2 theta, wherein the stable crystalline form of etoricoxib has no detectable quantity of any of form IV, form V or hemihydrate form of etoricoxib on storage at about 25°C and about 60% relative humidity for at least two months, as determined by absence of characteristic peak reflections at 8.7 and 15.2±0.2 degrees 2 theta for Form IV; by absence of characteristic peak reflections at 6.5 and 17.7±0.2 degrees 2 theta for Form V; by absence of characteristic peak reflections at 8.1±0.2 degrees 2 theta for the hemihydrate.

10. The stable crystalline form of etoricoxib as claimed in claim 9, **characterized by** PXRD pattern having peak reflections at 7.1, 9.7, 11.8, 15.5 and 22.7±0.2 degrees 2 theta, wherein the stable crystalline form of etoricoxib has no detectable quantity of any of form IV, form V or hemihydrate form of etoricoxib on storage at 40°C and 75% relative humidity for a period of about six months, as determined by absence of characteristic peak reflections at 8.7 and 15.2±0.2 degrees 2 theta for Form IV; by absence of characteristic peak reflections at 6.5 and 17.7±0.2 degrees 2 theta for Form V; by absence of characteristic peak reflections at 8.1±0.2 degrees 2 theta for the hemihydrate.

## Patentansprüche

1. Verfahren zur Herstellung von kristallinem Etoricoxib, **dadurch gekennzeichnet, dass** das PXRD-Muster Spitzenreflexionen bei 7,1, 9,7, 11,8, 15,5 und 22,7±0,2 Grad 2 Theta aufweist, umfassend:
a) Behandeln von Etoricoxib mit einem Lösungsmittelsystem, das mindestens ein Lösungsmittel umfasst, das aus der Gruppe ausgewählt ist, die aus Glykolen, Polyalkylenglykolen und Mischungen davon besteht; und
b) Isolieren des kristallinen Etoricoxib.

2. Verfahren nach Anspruch 1, wobei das Lösungsmittelsystem ein C1-C5-alkoholisches Lösungsmittel umfasst, wobei das Lösungsmittel aus der Gruppe ausgewählt ist, die aus Methanol, Ethanol, Isopropanol, n-Propanol, n-Butanol, Isobutanol, tert-Butanol und Mischungen davon besteht.

3. Verfahren nach Anspruch 1, wobei das Lösungsmittelsystem ein C1-C5-alkoholisches Lösungsmittel und Polyalkylenglykol im Volumenverhältnis von 0,25:550 bis 2,5: 170 umfasst.

4. Verfahren nach Anspruch 1, wobei die Behandlung das Kristallisieren von Etoricoxib aus einem Lösungsmittelsystem, das ein C1-C5-alkoholisches Lösungsmittel und Polyalkylenglykol umfasst, umfasst.

5. Verfahren nach Anspruch 1, wobei das erhaltene kristalline Etoricoxib ein charakteristisches PXRD-Muster aufweist, das Spitzenreflexionen bei 7,1, 9,7, 11,8, 15,5 und 22,7 ±0,2 Grad 2 Theta aufweist, und keine nachweisbare Menge einer aus der Form IV, Form V oder der Hemihydratform von Etoricoxib aufweist.

6. Verfahren nach Anspruch 1, wobei das erhaltene kristalline Etoricoxib ein charakteristisches PXRD-Muster aufweist, das Spitzenreflexionen bei 7,1, 9,7, 11,8, 15,5 und 22,7 ±0,2 Grad 2 Theta aufweist, wobei die stabile kristalline Form von Etoricoxib keine nachweisbare Menge der Form IV, festgestellt durch das Fehlen charakteristischer Spitzenreflexionen bei 8,7 und 15,2 ±0,2 Grad 2 Theta, aufweist.

7. Verfahren nach Anspruch 1, wobei das erhaltene kristalline Etoricoxib ein charakteristisches PXRD-Muster aufweist, das Spitzenreflexionen bei 7,1, 9,7, 11,8, 15,5 und 22,7 ±0,2 Grad 2 Theta aufweist, wobei die stabile kristalline Form von Etoricoxib keine nachweisbare Menge der Form V, festgestellt durch das Fehlen charakteristischer Spitzenreflexionen bei 13,7±0,2 Grad 2 Theta oder bei 6,5 und 17,7±0,2 Grad 2 Theta aufweist.

8. Verfahren nach Anspruch 1, wobei das erhaltene kristalline Etoricoxib ein charakteristisches PXRD-Muster aufweist, das Spitzenreflexionen bei 7,1, 9,7, 11,8, 15,5 und 22,7 ±0,2 Grad 2 Theta aufweist, wobei die stabile kristalline Form von Etoricoxib keine nachweisbare Menge der Hemihydratform, festgestellt durch das Fehlen charakteristischer Spitzenreflexionen bei 8,1 ±0,2 Grad 2 Theta, aufweist.

9. Stabile kristalline Form von Etoricoxib, die durch ein Verfahren nach einem der Ansprüche 1 bis 8 erhalten werden kann, **dadurch gekennzeichnet, dass** das PXRD-Muster Spitzenreflexionen bei 7,1, 9,7, 11,8, 15,5 und 22,7 ±0,2 Grad 2 Theta aufweist, wobei die stabile kristalline Form von Etoricoxib keine nachweisbare Menge einer aus der Form IV, Form V oder der Hemihydratform von Etoricoxib bei Lagerung bei etwa 25 °C und etwa 60 % relativer Feuchtigkeit über mindestens zwei Monate aufweist, festgestellt durch das Fehlen charakteristischer Spitzenreflexionen bei 8,7 und 15,2 ±0,2 Grad 2 Theta für Form IV; durch das Fehlen charakteristischer Spitzenreflexionen bei 6,5 und 17,7 ±0,2 Grad 2 Theta für Form V; durch das Fehlen charakteristischer Spitzenreflexionen bei 8,1 ±0,2 Grad 2 Theta für das Hemihydrat.

10. Stabile kristalline Form von Etoricoxib nach Anspruch 9, **dadurch gekennzeichnet, dass** das PXRD-Muster Spitzenreflexionen bei 7,1, 9,7, 11,8, 15,5 und 22,7 ±0,2 Grad 2 Theta aufweist, wobei die stabile kristalline Form von Etoricoxib keine nachweisbare Menge einer aus der Form IV, Form V oder der Hemihydratform von Etoricoxib bei Lagerung bei etwa 40 °C und etwa 75 % relativer Feuchtigkeit über einen Zeitraum von etwa sechs Monaten aufweist, festgestellt durch das Fehlen charakteristischer Spitzenreflexionen bei 8,7 und 15,2 ±0,2 Grad 2 Theta für Form IV; durch das Fehlen charakteristischer Spitzenreflexionen bei 6,5 und 17,7 ±0,2 Grad 2 Theta für Form V; durch das Fehlen charakteristischer Spitzenreflexionen bei 8,1 ±0,2 Grad 2 Theta für das Hemihydrat.

## Revendications

1. Procédé de préparation d'étoricoxib cristallin, **caractérisé en ce que** le diagramme de DRXP ayant des pics de diffraction à 7,1, 9,7, 11,8, 15,5 et 22,7 ± 0,2 degré 2 thêta comprenant :
a) le traitement de l'étoricoxib avec un système de solvants comprenant au moins un solvant sélectionné dans le groupe constitué par des glycols, des polyalkylène glycols et des mélanges de ceux-ci ; et
b) l'isolement de l'étoricoxib cristallin.

2. Procédé selon la revendication 1, dans lequel le système de solvants comprend un solvant alcoolique en C1 à C5 dans lequel le solvant est choisi dans le groupe constitué par le méthanol, l'éthanol, l'isopropanol, le n-propanol, le n-butanol, l'isobutanol, le butanol tertiaire et des mélanges de ceux-ci.

3. Procédé selon la revendication 1, dans lequel le système de solvants comprend un solvant alcoolique en C1 à C5 et du polyalkylène glycol selon le rapport de 0,25/550 en volume à 2,5/170 en volume.

4. Procédé selon la revendication 1, dans lequel le traitement comprend la cristallisation de l'étoricoxib à partir d'un système de solvants comprenant un solvant alcoolique en C1 à C5 et du polyalkylène glycol.

5. Procédé selon la revendication 1, dans lequel l'étoricoxib cristallin obtenu a un diagramme de DRXP caractéristique ayant des pics de diffraction à 7,1, 9,7, 11,8, 15,5 et 22,7 ± 0,2 degré 2 thêta et n'a pas de quantité détectable de l'une quelconque des formes parmi la forme IV, la forme V ou la forme d'hémihydrate de l'étoricoxib.

6. Procédé selon la revendication 1, dans lequel l'étoricoxib cristallin obtenu a un diagramme de DRXP caractéristique ayant des pics de diffraction à 7,1, 9,7, 11,8, 15,5 et 22,7 ± 0,2 degré 2 thêta, dans lequel la forme cristalline stable de l'étoricoxib n'a pas de quantité détectable de la forme IV comme est déterminé par l'absence de pics de diffraction caractéristiques à 8,7 et 15,2 ± 0,2 degré 2 thêta.

7. Procédé selon la revendication 1, dans lequel l'étoricoxib cristallin obtenu a un diagramme de DRXP caractéristique ayant des pics de diffraction à 7,1, 9,7, 11,8, 15,5 et 22,7 ± 0,2 degré 2 thêta, dans lequel la forme cristalline stable d'étoricoxib n'a pas de quantité détectable de la forme V comme est déterminé par l'absence de pics de diffraction caractéristiques à 13,7 ± 0,2 degré 2 thêta ou à 6,5 et 17,7 ± 0,2 degré 2 thêta.

8. Procédé selon la revendication 1, dans lequel l'étoricoxib cristallin obtenu a un diagramme de DRXP caractéristique ayant des pics de diffraction à 7,1, 9,7, 11,8, 15,5 et 22,7 ± 0,2 degré 2 thêta, dans lequel la forme cristalline stable d'étoricoxib n'a pas de quantité détectable de la forme d'hémihydrate comme est déterminé par l'absence de pics de diffraction caractéristiques à 8,1 ± 0,2 degré 2 thêta.

9. Forme cristalline stable d'étoricoxib pouvant être obtenue par un procédé selon l'une quelconque des revendications 1 à 8, **caractérisée par** un diagramme de DRXP ayant des pics de diffraction à 7,1, 9,7, 11,8, 15,5 et 22,7 ± 0,2 degré 2 thêta, dans lequel la forme cristalline stable de l'étoricoxib n'a pas de quantité détectable de l'une quelconque des formes parmi la forme IV, la forme V ou la forme d'hémihydrate de l'étoricoxib pendant un stockage à environ 25 °C et environ 60 % d'humidité relative pendant au moins deux mois, comme est déterminé par l'absence de pics de diffraction caractéristiques à 8,7 et 15,2 ± 0,2 degré 2 thêta pour la forme IV ; par l'absence de pics de diffraction caractéristiques à 6,5 et 17,7 ± 0,2 degré 2 thêta pour la forme V ; par l'absence de pics de diffraction caractéristiques à 8,1 ± 0,2 degré 2 thêta pour l'hémihydrate.

10. Forme cristalline stable d'étoricoxib selon la revendication 9, **caractérisée par** un diagramme de DRXP ayant des pics de diffraction à 7,1, 9,7, 11,8, 15,5 et 22,7 ± 0,2 degré 2 thêta, dans lequel la forme cristalline stable de l'étoricoxib n'a pas de quantité détectable de l'une quelconque des formes parmi la forme IV, la forme V ou la forme d'hémihydrate de l'étoricoxib pendant un stockage à environ 40 °C et environ 75 % d'humidité relative pendant une période d'environ six mois, comme est déterminé par l'absence de pics de diffraction caractéristiques à 8,7 et 15,2 ± 0,2 degré 2 thêta pour la forme IV ; par l'absence de pics de diffraction caractéristiques à 6,5 et 17,7 ± 0,2 degré 2 thêta pour la forme V ; par l'absence de pics de diffraction caractéristiques à 8,1 ± 0,2 degré 2 thêta pour l'hémihydrate.
